(19) Europäisches Patentamt
European Patent Office
Office européen des brevets

(11) **EP 4 336 175 B1**

(12) **EUROPEAN PATENT SPECIFICATION**

(45) Date of publication and mention
of the grant of the patent:
**03.09.2025 Bulletin 2025/36**

(21) Application number: **21940635.2**

(22) Date of filing: **17.05.2021**

(51) International Patent Classification (IPC):
**G01N 23/203** *(2006.01)* **G01N 23/2251** *(2018.01)*
**G01N 33/2028** *(2019.01)*

(52) Cooperative Patent Classification (CPC):
**G01N 23/203; G01N 23/2251; G01N 33/2028;**
G01N 2223/053; G01N 2223/60

(86) International application number:
**PCT/ES2021/070350**

(87) International publication number:
**WO 2022/243576 (24.11.2022 Gazette 2022/47)**

(54) **METHOD FOR THE QUANTITATIVE ASSESSMENT OF THE AREA AFFECTED BY THE TRANSFORMATION OF FERRITE IN DUAL-PHASE STEELS**

VERFAHREN ZUR QUANTITATIVEN BEURTEILUNG DER DURCH DIE UMWANDLUNG VON FERRIT IN ZWEIPHASIGE STÄHLE BETROFFENEN FLÄCHE

PROCÉDÉ POUR L'ÉVALUATION QUANTITATIVE DE LA ZONE AFFECTÉE PAR LA TRANSFORMATION DE LA FERRITE EN ACIERS DUAUX DP

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.03.2024 Bulletin 2024/11**

(73) Proprietor: **Asociacion Centro Tecnologico Ceit 20018 San Sebastian (Gipuzkoa) (ES)**

(72) Inventors:
• **IZA MENDIA, Amaia**
20018 SAN SEBASTIAN (GIPUZKOA) (ES)
• **JORGE BADIOLA, Denis**
20018 SAN SEBASTIAN (GIPUZKOA) (ES)
• **GUTIERREZ SANZ, Isabel**
20018 SAN SEBASTIAN (GIPUZKOA) (ES)

(74) Representative: **Evens, Paul Jonathan et al Maguire Boss
24 East Street
St. Ives, Cambridgeshire PE27 5PD (GB)**

(56) References cited:
**CN-A- 101 929 964     CN-A- 109 959 670**

• **ISASTI NEREA ET AL: "Analysis of Complex Steel Microstructures by High-Resolution EBSD", JOM: JOURNAL OF METALS, SPRINGER NEW YORK LLC, UNITED STATES, vol. 68, no. 1, 26 October 2015 (2015-10-26), pages 215 - 223, XP035949050, ISSN: 1047-4838, [retrieved on 20151026], DOI: 10.1007/S11837-015-1677-0**
• **ISASTI NEREA ET AL.: "Analysis of Complex Steel Microstructures by High-Resolution EBSD", JOM : JOURNAL OF METALS, vol. 68, no. 1, 26 October 2015 (2015-10-26), New York Llc, United States, pages 215 - 223, XP035949050, ISSN: 1047-4838, [retrieved on 20211203], DOI: 10.1007/s11837-015-1677-0**
• **ANDO REON, MATSUNO TAKASHI, MATSUDA TOMOKO, YAMASHITA NORIO, YOKOTA HIDEO, GOTO KENTA, WATANABE IKUMU: "Analysis of nano-hardness distribution near the ferrite-martensite interface in a dual phase steel with factorization of its scattering behavior", TETSU TO HAGANE: JOURNAL OF THE IRON AND STEEL INSTITUTE OF JAPAN, IRON AND STEEL INSTITUTE OF JAPAN. TOKYO., JP, vol. 106, no. 12, 1 January 2020 (2020-01-01), JP , pages 944 - 952, XP093009671, ISSN: 0021-1575, DOI: 10.2355/tetsutohagane.TETSU-2020-037**

EP 4 336 175 B1

# Description

## Sector of the art

The present invention belongs to the field of metallurgy, more specifically to the field of microscopy metallurgical characterisation, and relates to a method for characterising ferrite-martensite dual-phase steels, specifically for identifying and quantifying the ferrite area affected by martensite transformation in dual-phase steels, by means of the EBSD microscopy technique. The invention also relates to the use of said quantification of TAF (Transformation Affected Ferrite, i.e., ferrite affected by transformation) areas in mechanical property prediction models.

## Prior Art

Dual-phase steels are steels with a resulting ferrite-martensite microstructure at room temperature. The way for obtaining these steels is through intercritical annealing which produces, in this high-temperature stage, a ferrite-austenite microstructure before final cooling. After a quick cooling or tempering, austenite transforms into martensite, obtaining the ferrite-martensite dual-phase structure of the final steel.

As a result of the transformation of austenite into martensite (M) in DP (dual-phase) steels, the ferrite adjacent to the martensite hardens. The authors of the invention referred to these ferrite areas affected by the transformation of pre-existing austenite into martensite as "TAF areas". The formation of TAF areas is limited to the generation of small local misorientations in ferrite adjacent to martensite.

The origin of the hardening of the ferrite areas (TAF areas) adjacent to martensite lies in the volumetric change caused by the transformation of those austenite areas (high-temperature phase) into martensite (low-temperature phase) during cooling after annealing. Expansion occurs in martensite, which causes the plastic deformation of the adjacent ferrite, giving rise to TAF areas.

Thus, these TAF areas have characteristics different from those of conventional ferrite, such as greater hardness. For this reason, it is important to characterise said hardened TAF areas, for example, in order to correctly predict the mechanical properties of DP steels, and thereby cover a current need in the state of the art.

Nanoindentation is used in the state of the art for characterising the mechanical properties of ferrite-martensite steels. However, nanoindentation presents the limitation that nanohardness measurements are affected by the proximity of the location of the indentation to the ferrite-martensite, F/M, interface, which results in certain spatial limitation, particularly below the surface, where the position of the interface is unknown, with this being able to lead to the attainment of local hardness values that are not sufficiently representative of the sample.

In the state of the art there are also micromechanical approaches for predicting the mechanical behaviour of DP steels. However, direct application of these models to DP steels has given rise to significant deviations between the experimental results and the predictions. One of the factors which is put forward as a determining factor in the discrepancies observed is that which corresponds to ferrite hardening effect resulting from the transformation of austenite into martensite during the cooling of the steel, i.e., the formation of hardened ferrite areas (TAF areas), which are often overlooked by existing micromechanical models. As explained, martensite, through its formation mechanism, induces plastic deformation in ferrite, hardening it near the ferrite-martensite (FM) interface during cooling. This phenomenon contributes to additional hardening of the steel that is often omitted.

For this reason, it is important to characterise the transformation affected ferrite areas (TAF), which is a complex process. However, in the current state of the art there is no methodology capable of characterising TAF areas, i.e., identifying and quantifying them, or in other words, differentiating between different states of ferrite, specifically between soft ferrite and hard ferrite (TAF) in DP steels.

In order to identify and quantify TAF areas, the present invention contemplates using the EBSD technique, associated with scanning electron microscopy.

The EBSD (Electron Backscatter Diffraction) technique is an accessory attached to a scanning electron microscope which allows identifying the phases of a crystalline material (for example, a metal such as steel), as well as misorientations between the crystals of the phases present in said material, both for single-phase materials and for multiphase materials. First, after preparing the sample, an area of the sample to be measured, which is divided into pixels of the size chosen by the user, is selected, with a scan being performed in said area irradiating the area of each pixel with an electron beam. A misorientation is assigned to each pixel based on the Kikuchi pattern that the electron beam from said pixel of the sample projects onto a phosphorescent detection screen. The use of EBSD in a field emission scanning electron microscope is a fast, reliable, and very high-resolution method to obtain data on crystalline structures and their 3-dimensional orientation.

Once the misorientation data for each pixel of the area has been obtained by means of EBSD, it is processed by means of data processing software, with there being a multitude of software for processing data obtained by means of EBSD such as, for example, the commercial software TSL-OIM. Thus, the data obtained by means of EBSD can be processed with any of the data processing software adapted to that end.

To date, to the knowledge of the applicant of the present invention, the EBSD technique has not been used in a systematic and structured manner for defining and quantifying hardened ferrite areas (TAF) affected by the adjacent martensite. In fact, there is no methodology established in the state of the art capable of differentiating between ferrite, specifically between soft ferrite and hard ferrite (TAF), which will be referred to hereinafter precisely as ferrite subphases.

Patent documents on methods for the quantitative measurement of microstructural characteristics in steels by means of the EBSD technique, as well as scientific publications which analyse the ferrite-martensite interface by means of EBSD, are known in the state of the art; but none of them disclose or suggest a methodology for identifying and quantifying TAF areas.

Some of these already known patent documents and scientific publications are listed below:
CN102735703A**:** Method for quantitatively evaluating retained austenite in steel by EBSD (electron back scattering diffraction).

CN103454294A**:** Method for quantitatively evaluating each phase structure in hot rolling TRIP (transformation-induced plasticity) steel.

CN105203438A**:** Determination method for austenitic grain size of pearlite type wire rod.

ISATI NERA ET AL: "Analysis of Complex Steel Microstructures by High-Resolution EBSD", JOM: JOURNAL OF METALS, SPRINGER NEW YORK LLC, UNITED STATES, vol. 68, no. 1, 26 October 2015 (2015-10-26), pages 215-223
The present invention seeks to overcome the preceding limitations of the state of the art by providing a robust and precise methodology for characterising hardened ferrite areas (TAF areas) affected by martensite in ferrite-martensite dual-phase steels, using to that end the EBSD technique. This methodology will allow identifying and quantifying TAF areas.

**Object of the invention**

The object of the present invention relates to a method for characterising a steel sample. Specifically, it relates to a method for identifying and quantifying, based on the attainment and processing of data obtained by means of EBSD, TAF areas in recrystallised DP (ferrite-martensite) steels, by means of differentiating ferrite between its soft ferrite and hardened ferrite (TAF areas) sub-phases.

These TAF areas correspond to the ferrite areas which are hardened by the adjacent martensite as a result of the transformation of austenite into martensite. For this reason, it is important to quantify said hardened TAF areas, for example, in order to correctly predict the mechanical properties of ferrite-martensite DP steels, thereby covering a current need in the state of the art.

Therefore, the invention is capable of identifying and quantifying TAF areas in recrystallised dual-phase steels by means of EBSD, a technique which has never been used before to that end. This is achieved by means of the identification of ferrite and martensite phases in the pixels of an EBSD scan carried out on the recrystallised dual-phase steel sample, and by means of a suitable selection, according to the invention, of thresholds of the Kernel Average Misorientation (KAM) parameter, during the analysis of data obtained by means of EBSD, which is a parameter conventionally used for EBSD data.

The KAM parameter has never been used to differentiate sub-phases of one and the same phase in the state of the art, for example, to differentiate ferrite between soft ferrite and hardened ferrite (TAF areas), like in the case of the present invention. This is because, previously, being the same phase (ferrite), it is not expected that soft ferrite can be differentiated from hardened ferrite (TAF areas) affected by adjacent martensite transformation by means of EBSD, since both of them have the same Kikuchi pattern characteristics of their crystalline structure and orientation as they are both ferrite, and they would be conventionally identified as ferrite by means of EBSD. For this reason, the EBSD technique would not be contemplated for quantifying TAF, since said technique is not capable of differentiating between ferrite sub-phases previously.

The characterisation method for characterising a recrystallised dual-phase steel sample of the present invention comprises identifying the TAF areas of the sample, by means of carrying out the following steps a-e):

- a) carrying out at least one EBSD scan of a surface area of the sample to identify ferrite pixels and martensite pixels;
- b) calculating a KAM deviation with respect to n neighbours for the ferrite pixels;
- c) assigning as a TAF area the ferrite pixels adjacent to at least one martensite pixel;
- d) additionally assigning as a TAF area the ferrite pixels adjacent to at least one ferrite pixel assigned as a TAF area and which also have a KAM deviation with respect to the n-neighbours thereof comprised in a range $[\alpha, \beta]$, where $\alpha \geq 0.3°$ and $\beta \leq 2.0°$;
- e) iterating step d) with respect to all the ferrite pixels adjacent to those previously assigned as a TAF area.

For step c), optionally and preferably, the additional condition of said ferrite pixels adjacent to the martensite having a KAM deviation with respect to the n-neighbours thereof comprised in a range $[\alpha, \beta]$, where $\alpha \geq 0,3°$ and $\beta \leq 2,0°$, is also imposed on the condition of assigning as a TAF area the ferrite pixels adjacent to at least one martensite pixel.

The first step a) consists of identifying the ferrite areas based on carrying out at least one EBSD scan on the sample. Once data corresponding to ferrite is identified (step a), the inventors according to the method of the present invention have carried out steps b-e) mentioned above for detecting the hardened ferrite sub-phase (TAF areas) in the ferrite phase.

Steps a-e) of the characterisation method of the present invention will be explained in more detail below in the detailed description of the invention.

Thus, according to the characterisation method for characterising dual-phase steels of the present invention, a ferrite pixel will be identified as a "TAF area" if it complies with one of the following two criteria:

- The first criterion is that the TAF area must be ferrite adjacent to martensite, i.e., a ferrite-martensite interface area, i.e., ferrite pixels adjacent to martensite pixels. Optionally, the additional condition of the pixels having to have KAM values with respect to their neighbours of between 0.3 and 2° is imposed.
- The second criterion is that the pixels identified as TAF areas, in addition to being adjacent to the pixels identified previously as TAF areas, must have KAM values with respect to their neighbours of between 0.3 and 2°.

This second criterion refers to the "Kernel Average Misorientation" (KAM) parameter. This is a preestablished parameter that is known in the scientific field which uses the EBSD technique and allows quantifying the misorientation between pixels. However, neither the KAM parameter nor the definition of its ranges has been used previously for identifying and quantifying hardened ferrite (TAF) in DP steels.

After applying the method for characterising a recrystallised ferrite-martensite dual-phase steel sample of the present invention, the pixels of the characterised sample corresponding to the 3 phases, specifically the martensite phase and the soft ferrite and hardened ferrite (TAF areas) sub-phases, will be identified. For this reason, it is then possible to mathematically calculate the percentage or fraction of each phase, and therefore the percentage of the hard phase of this steel, which would be the martensite phase plus the hardened ferrite phase (TAF areas), accordingly. The soft phase will be, therefore, the remaining soft ferrite phase, also being quantified.

Any conventional mathematical data selection and quantification method can be used to quantify the 3 phases once the pixels corresponding to each phase have been identified.

An example of a mathematical quantification method according to the invention consisting of several data post-processing steps will be provided below in the detailed description of the invention.

In summary, according to the present invention, the use of the EBSD technique and subsequent data processing allows:

- Differentiating and quantifying the two existing phases: ferrite (F) and martensite (M), which is already known.
- Identifying and quantifying ferrite sub-phases into soft ferrite and hardened ferrite (TAF areas), the latter being the novel part of the invention. The TAF areas are characterised according to the invention by means of those pixels adjacent to martensite pixels, or as those ferrite pixels adjacent to previously identified TAF pixels and additionally present orientation gradients caused by the adjacent martensite expressed by means of the KAM parameter, with a range of between 0.3 and 2°.

Another aspect of the present disclosure includes application of the quantification of TAF areas (hardened ferrite) in conventional tools for predicting the mechanical properties of DP steels of the state of the art, for the simulation of the micromechanical behaviour of said steels; steels widely used in the form of sheet metal in the automotive industry.

**Description of the Figures**

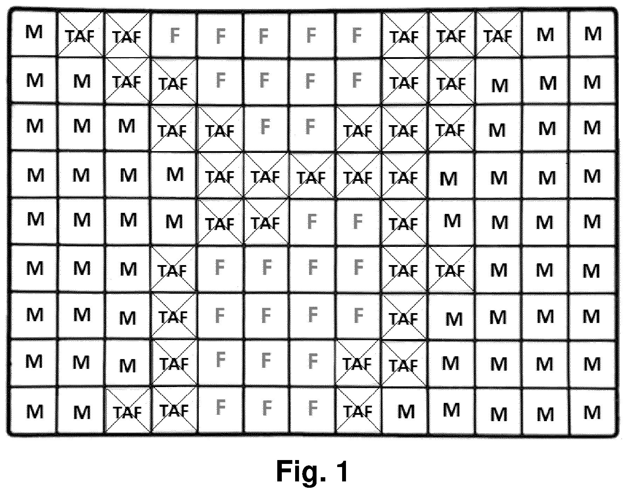

Figure 1 visually and schematically shows an example of a pixel scan such as that which would be carried out by means of the EBSD technique using the method for characterising a recrystallised dual-phase steel sample of the present invention, in which the pixels of the DP (ferrite - martensite) steel sample are identified according to the method of the invention as martensite (M), soft ferrite (F), and hardened ferrite or TAF (TAF).

**Detailed description of the invention**

The present invention describes a method for characterising a steel sample. More specifically, it relates to a method for identifying and quantifying the hardened ferrite area (TAF areas) in recrystallised DP steels which contain ferrite and martensite, by means of differentiating ferrite between its soft ferrite and hardened ferrite (TAF areas) sub-phases, using to that end the EBSD technique ("Electron Backscatter Diffraction technique").

These TAF areas correspond to the ferrite areas which are hardened by the adjacent martensite as a result of the transformation of austenite into martensite. For this reason, it is important to quantify said hardened TAF areas, for example, in order to correctly predict the mechanical properties of DP steels, thereby covering a current need in the state of the art.

This is achieved by means of carrying out at least one EBSD scan of suitable step size on the recrystallised DP steel sample in order to obtain data, and by means of suitably selecting thresholds in the Kernel parameter (KAM) during the data analysis step, which is a parameter the use of which is known for the EBSD technique.

More specifically, the invention relates to a method for characterising a recrystallised dual-phase steel sample, which comprises identifying the TAF areas of the sample, comprising the following steps:

- a) carrying out at least one EBSD scan of a surface area of the sample to identify ferrite pixels and martensite pixels;

- b) calculating a KAM deviation with respect to n neighbours for the ferrite pixels;
- c) assigning as a TAF area the ferrite pixels adjacent to at least one martensite pixel;
- d) additionally assigning as a TAF area the ferrite pixels adjacent to at least one ferrite pixel assigned as a TAF area in the previous step and which also have a KAM deviation with respect to n neighbours comprised in a range [α, β], where α ≥ 0.3° and β ≤ 2.0°;
- e) iterating step d) with respect to all the ferrite pixels adjacent to those previously assigned as a TAF area.

For step c), optionally and preferably, the additional condition of said ferrite pixels adjacent to the martensite having a KAM deviation with respect to the n-neighbours thereof comprised in a range [α, β], where α ≥ 0,3° and β ≤ 2,0°, is also imposed on the condition of assigning as a TAF area the ferrite pixels adjacent to at least one martensite pixel.

Surprisingly, it has been observed that not all the ferrite areas adjacent to martensite are necessarily affected by the adjacent martensite; i.e., they are not necessarily TAF areas. Thus, imposing the additional condition of said ferrite pixels adjacent to the martensite presenting a KAM of between 0.3 and 2° results in more optimal values in terms of the identification of ferrite pixels as TAF areas.

The method for characterising TAF areas of the invention by means of EBSD is described in more detail below step by step.

1) Carrying out at least one EBSD scan to identify ferrite pixels and martensite pixels (step a):

The first feature characteristic of the present invention is carrying out at least one scan to obtain data by means of EBSD, preferably by means of a fine scan step, to thereby enable measuring the ferrite areas affected by martensite transformation (TAF areas). This fine scan can preferably be carried out after a prior larger scan step, or independently of same.

The fine scan step according to the present invention will be less than or equal to a 200 nm, preferably less than or equal to 100 nm, more preferably less than 60 nm, and even more preferably between 50 and 60 nm.

In fact, the inventors have concluded that a fine step size greater than 60 nm results in a less suitable characterisation of the TAF areas, so values less than 60 nm are more preferred. It appears that a sufficiently fine step size (i.e., at least 60 nm) allows analysing the surroundings or boundaries of martensite and ferrite grains in greater detail, where a more accurate phase identification and quantification can thus be obtained. By way of non-limiting example, a fine scan step of 50 nm, covering an area of, for example, $10^6$ pixels (50 μmax 50 μm), could be used. However, resolution of two Kikuchi patterns under standard microscopy conditions at positions closer to or below 50 nm is currently not possible due to the spatial limitation of the technique. The inventors understand that it may be possible to resolve said patterns at smaller distances in the future, for example, due to improvements in the EBSD technique itself such as the EBSD-based TKD ("Transmission Kikuchi Diffraction") technique, and for this reason step sizes less than 50 nm are contemplated in the present invention.

The additional use of at least a first larger step scan before carrying out the fine step scan is optional and preferred for carrying out the present invention. The larger step size would not be limited, but it will preferably be equal to or greater than 60 nm, for example 200 nm. Carrying out at least one additional scan of a greater size allows obtaining a first characterisation of the microstructure of the sample to thereby place same in the microstructure of the sample and locate the ferrite-martensite interface of interest for subsequent detailed study with a fine size step for the purpose of identifying and quantifying the hardened ferrite areas surrounding martensite. The optimal greater step size will be determined based on the size of the steel microstructure to be analysed. Thus, it will preferably be that which allows complying with the following 2 characteristics for the specific steel microstructure to be observed: 1) defining the grains with sufficient resolution within the scan, allowing ferrite grain size to be clearly distinguished from martensite grain size; and 2) additionally covering a representative and significant area of the microstructure.

According to the present invention, scans with different step sizes can be carried out. Preferably, scan with a larger step size will be carried out for measuring the grain size of the microstructure and including all the phases of the steel, and another subsequent fine size scan for measuring at higher resolution the hardened ferrite areas (TAF areas) affected by the transformation of adjacent martensite. It is contemplated to carry out several larger and fine step scans.

Given that both ferrite pixels and martensite pixels have been obtained in the scan, first the ferrite pixels must be selected by means of partitioning the data corresponding to the martensite phase. This selection can be carried out, for example, by means of commercial software, such as the TSL-OIM software.

Areas corresponding to the ferrite phase and the martensite phase can be separated conventionally through the IQ ("Image Quality") parameter for the quality of Kikuchi pattern, since both phases produce different characteristic Kikuchi patterns, allowing the identification thereof in ferrite or martensite. Differentiation between the ferrite and martensite phase by means of the EBSD technique is conventional and has been used for a long time (numerous publications indicate same).

Martensite is differentiated by its size and the quality of the Kikuchi patterns. Specifically, martensite and ferrite are differentiated by:

a. The grain size (set of pixels which form a ferrite or martensite unit), which is smaller in the case of martensite than in ferrite in steels of this type; and

b. Quality of the Kikuchi lines (worse in martensite than in ferrite).

Thus, after this partition, the martensite phase can be separated from the data, such that the resulting data set only includes ferrite; specifically its 2 sub-phases, i.e., both the soft ferrite and the hardened ferrite (TAF areas).

It should be taken into account that, in this step, ferrite sub-phases, such as soft ferrite or TAF areas, have yet to be identified in the scan itself. Differentiation of the ferrite sub-phases between soft ferrite or hardened ferrite (TAF) will only take place in the subsequent analysis of the obtained data by means of EBSD data analysis software. This software can be incorporated in the EBSD data obtaining software itself or can be independent of same. The use of any conventional EBSD data processing software such as, for example, the commercial software TSL-OIM, is contemplated.

This identification of ferrite sub-phases will be carried out according to steps b)-e) which will be explained below.

2) Identifying TAF areas of the ferrite phase (steps b-e):

After carrying out step a), ferrite pixels can then be separated into their soft ferrite and hardened ferrite (TAF) sub-phases. To that end, commercial software, such as the TSL-OIM software, or software adapted for that purpose, will be used.

To that end, the following steps b-e) will be carried out:

- b) calculating a KAM deviation with respect to n neighbours for the ferrite pixels;
- c) assigning as a TAF area the ferrite pixels adjacent to at least one martensite pixel:
- d) additionally assigning as a TAF area the ferrite pixels adjacent to at least one ferrite pixel assigned as a TAF area in the previous step and which also have a KAM deviation with respect to n neighbours comprised in a range $[\alpha, \beta]$, where $\alpha \geq 0.3°$ and $\beta \leq 2.0°$;
- e) iterating step d) with respect to all the ferrite pixels adjacent to those previously assigned as a TAF area.

For step c), optionally and preferably, the additional condition of said ferrite pixels adjacent to the martensite having a KAM deviation with respect to the n-neighbours thereof comprised in a range $[\alpha, \beta]$, where $\alpha \geq 0,3°$ and $\beta \leq 2,0°$, is also imposed on the condition of assigning as a TAF area the ferrite pixels adjacent to at least one martensite pixel.

First, in the already separated data corresponding to ferrite, those ferrite pixels immediately adjacent to the martensite pixels are defined as TAF areas, without imposing any condition of KAM value on these first pixels. This corresponds to step c) of the method of the present invention.

Next, those pixels adjacent to the TAF areas previously identified in step c) that additionally present a kernel average misorientation (KAM) of between 0.3 and 2° will also be identified as TAF areas. In other words, in addition to the condition of being adjacent to a previously defined TAF area, the condition of complying with the condition of presenting a KAM of less than or equal to 2° is imposed on the pixels not immediately adjacent to the ferrite-martensite interface in order to be defined as a TAF area. This corresponds to step d) of the method of the present invention.

The calculation of KAM deviation, i.e., step b), can be carried out at any time before step c) or d).

By means of the KAM parameter, the TAF areas, i.e., the ferrite areas adjacent to newly formed martensite and the hardness of which is affected by the new martensite, are detected and quantified. This selection of a KAM value of between 0.3 and 2° is a specific feature of the present invention. Furthermore, the KAM value of each pixel is calculated with respect to the neighboring pixels, preferably according to this invention with respect to neighboring third parties, that is, with respect to three pixels away in all directions from the study ferrite pixel.

Lastly, step e); i.e., step d) will be iterated with respect to all the ferrite pixels adjacent to those previously assigned as a TAF area, will be carried out. This will also identify all those ferrite pixels adjacent to other previously identified TAF areas as TAF areas, provided that they additionally present KAM deviations comprised between 0.3 and 2°. This step e) of iterating step d) allows identifying all the TAF areas present in the sample.

Pixels not identified as TAF areas will therefore be identified as conventional or soft ferrite.

Therefore, according to the method for characterising dual-phase steels defined above by the inventors of the present invention, for the detection of the hardened ferrite sub-phase (TAF areas) in the ferrite phase, a ferrite area will be identified as a "TAF area" if it complies with one of the following two criteria:

- The first criterion is that the TAF area must be ferrite adjacent to martensite, i.e., a ferrite-martensite interface area, i.e., ferrite pixels adjacent to martensite pixels. Optionally and preferably, the additional condition of said ferrite pixels adjacent to the martensite presenting a KAM of between 0.3 and 2° is imposed on the first criterion.
- The second criterion is that the pixels identified as TAF areas, in addition to being adjacent to the pixels previously identified as TAF areas, must have KAM values with respect to their neighbours of between 0.3 and 2°.

Defining the condition of the first criterion of the TAF areas being ferrite areas adjacent to martensite pixels or pixels previously identified as TAF areas is important, since the interior of ferrite grains themselves, as a result of their own deformation and of not being a perfect crystal, may present deviations within the grain between pixels, where the case in which they present average misorientations with KAM values of up to 2° may arise, and for this reason these pixels are erroneously identified as TAF areas. This definition (being adjacent) prevents the possibility of erroneously defining soft ferrite areas as hardened ferrite TAF.

The second criterion comprises the pixel presenting a value of the "Kernel Average Misorientation (KAM)" parameter of between 0.3 and 2°. The KAM parameter is a preestablished parameter that is known in the scientific field which uses the EBSD technique and allows quantifying the misorientation between pixels. However, neither the KAM parameter nor the definition of its ranges has been used previously for identifying and quantifying phases or sub-phases such as, for example, the hardened ferrite (TAF areas) of the present invention. The definition of TAF areas as ferrite areas with average misorientation KAM of between 0.3 and 2° is a feature characteristic of the invention, and allows characterising the TAF areas of dual-phase steels by means of the EBSD technique.

The KAM parameter, according to its definition, calculates the mean or average misorientation of a point or pixel with respect to all its neighbours, defining each of them by its misorientation angle.

Kernel Average Misorientation (KAM) is a measurement of the local misorientation of the grain which is generally derived from EBSD data. To formally define the KAM, $o_{I,jj,jj}$ denotes the orientation in the position of pixel $(i,j)$ and $N(i,j)$ denotes the set of all neighbouring pixels. Kernel Average Misorientation $(KAM)_{i,j}$ in the position of pixel $(i,j)$ is given by:

$$KAM_{i,j} = \frac{1}{|N(i,j)|} \sum_{(k,l)\in(i,j)} \omega(o_{i,j}, o_{k,l})$$

In this case, $|N(i,j)|$ refers to the number of all neighboring pixels taken into consideration and the misorientation angle $\omega(o_{i,j}, o_{k,l})$ between orientation $o_{i,j}$ in the center $(i,j)$ and neighboring orientation $o_{k,l}$ corresponding to pixel $(k,l)$. The specific choice of set $N(i,j)$ of neighbouring pixels is crucial for calculating KAM. Additional criteria are usually imposed for calculating same:

- taking into consideration neighbours up to order n, for example, n = 1, 2, 3, etc.
- taking into consideration only neighbours with a misorientation angle smaller than a threshold KAM angle.

The preferred criterion imposed on n for this invention is n equal to 3 (measurement of misorientation with respect to up to 3 neighbouring pixels, or third neighbours), and ferrite pixels with KAM values of between 0.3 and 2° would be mandatorily selected. Thus, the criterion of n = 3 is preferred, where the present invention can be carried out by selecting other integer multiples of n (number of neighbours). In fact, it has been observed that, although choosing a high value of n improves the precision in KAM calculation as it is performed with respect to a larger number of pixels, on the other hand since these neighbouring pixels are increasingly farther from one another as n or the number of neighbours increases, the KAM values are less representative of the immediate surroundings of the pixel under study. To that end, there is a compromise in choosing the value n so as to optimise precision in calculating the KAM of the pixel under study. It has been observed that a value n = 3 allows obtaining more optimum KAM values.

According to the present invention, the KAM for a given point or the average misorientation of that point with all its neighbours (preferably up to the third neighbours in this invention), is calculated with the condition that the misorientations which exceed the imposed tolerance value (maximum misorientation of 2° and minimum misorientation of 0.3° in this invention) are excluded from the average calculation. Therefore, ferrite pixels adjacent to pixels previously identified as TAF areas that exceed a KAM of 2° or present a KAM of less than 0.3° will not be identified as TAF areas.

Mathematically, according to the characterisation method of the present invention, those ferrite pixels which comply with one of the following conditions will be defined as hardened ferrite areas (TAF areas):

1) Being a ferrite pixel the position of which is $(i\pm1, j\pm1)$ with respect to a martensite pixel $(i,j)$; i.e., a ferrite pixel adjacent to a martensite pixel, and Optionally and preferably, said pixel furthermore satisfying the condition of a KAM being comprised between a minimum angle and a maximum angle or KAM range $[\alpha, \beta]$, where $\alpha \geq 0.3°$ and $\beta \leq 2.0°$; with respect to its neighbours.

2) Being a ferrite pixel the position of which is $(i\pm1, j\pm1)$ with respect to a pixel previously defined as a TAF $(i,j)$; i.e., a ferrite pixel adjacent to a pixel of a TAF area, and said pixel furthermore satisfying the condition of a KAM being comprised between a minimum angle and a maximum angle or KAM range $[\alpha, \beta]$, where $\alpha \geq 0.3°$ and $\beta \leq 2.0°$; with respect to its neighbours, preferably with respect to its third neighbours for a step size comprised between 50 and 60 nm.

In a preferred embodiment, KAM will be calculated with respect to its third neighbours for scan (or step) sizes of 50-60 nm.

In the case of an upper KAM limit of 2°, it is an upper limit imposed on the KAM parameter that no pixel of the scan can exceed. Its choice is based on the fact that the 2° limit the misorientation from which the minimum microstructural unit known as subgrain is defined. The formation of a subgrain, understood as the crystallographic unit which is limited by misorientations of between 2 and 15°, could not be justified as resulting from the effect of martensite transformation, and for this reason 2° is defined as the upper limit. In other words, the formation of different subgrains is characteristic of metallic microstructures, even single-phase soft ferrite microstructures. For this reason, the subgrains bordering the hardened ferrite or TAF subgrains could be either soft ferrite or hardened ferrite, so they cannot be unequivocally established as TAF areas by means of the present method; and for this reason, said bordering subgrains are excluded from the calculation.

In the case of the lower KAM limit of 0.3°, its selection is based on the fact that it has been observed that it determines an angular threshold below which noise affects the measurement, and it is also based on the observation that the TAF area, as a result of being affected by martensite, has small misorientations in its structure itself. Noise depends on several factors, including sample preparation, the conditions established for signal acquisition, and even the abrupt incorporation as TAF of a large number of pixels in the recrystallised ferrite.

Thus, after carrying out data analysis and up to this point, the areas measured by means of EBSD of all 3 phases present in the sample have been identified as: martensite, soft ferrite, and hardened ferrite (TAF) areas. This identification of each of the pixels as belonging to one of the 3 preceding phases is visually and schematically depicted by way of example in Figure 1.

Optionally, if there is an interest to determine the subgrain size of any of the phases, the method includes defining a minimum angular tolerance of 2° for determining the subgrain size during the data analysis phase. This limit of 2° defines the subgrain boundary (and therefore delimits the area that constitutes a subgrain) taking into consideration that the misorientation between contiguous points is greater than 2°. This limit is imposed by the patent applicant, and is an upper limit imposed on the KAM parameter that no pixel in the scan must exceed in order to be considered as belonging to the subgrain. Its choice is based on the fact that the 2° limit the misorientation from which the minimum microstructural unit known as subgrain is defined. The subgrain boundaries can thereby be defined as those with misorientation greater than 2° between contiguous points or pixels, and the subgrain area can thereby be established.

It should be noted that this method can only be applied for the identification of TAF areas in recrystallised (deformation-free) and subsequently tempered dual-phase steel, which is what generally obtained in these steels. On the contrary, if the steel was mechanically deformed after martensite transformation resulting from tempering, new internal misorientations would be introduced, and the grain and subgrain boundaries would be reorganized, so that the criterion of maximum KAM threshold of 2° would no longer be useful for identifying and separating ferrite TAF areas from soft ferrite areas. Therefore, this invention would apply exclusively to dual-phase (ferrite-martensite) and recrystallised steels, for example, obtained through annealing and subsequent cooling during which the transformation of austenite into martensite occurs, with the resulting steel being dual-phase (ferrite-martensite) steel, and without mechanical deformation being applied to the steel before characterising it by means of the method of the present invention.

This method is novel and allows something unexpected until now, i.e., the detection by means of a microscopy technique such as EBSD of ferrite sub-phases which are previously indistinguishable microscopically or crystallographically as they are both ferrite.

3) Quantifying the phases:

Once the preceding criteria have been applied, the sample pixels corresponding to the 3 phases, specifically the martensite phase and the soft ferrite and hardened ferrite (TAF areas) subphases, are successfully identified. This identification of each of the pixels as belonging to one of the 3 preceding phases is visually and schematically depicted by way of example in Figure 1.

For this reason, the area percentage or fraction of each phase or subphase can then be calculated mathematically, and therefore, the area fraction of the hard phase of this steel (which would be martensite phase plus hardened ferrite phase or TAF areas) and that of the soft phase (which would be soft ferrite) can also be calculated accordingly.

Any conventional mathematical data selection and quantification method can be used to quantify the 3 phases once the pixels corresponding to each of the phases have been identified.

An example of a mathematical quantification method according to the invention would consist of the following data post-processing steps:

    a. Selecting data corresponding to martensite and ferrite grains and calculating the total area fraction of martensite by subtracting the area fraction of ferrite.
    b. For ferrite grains, partitioning the data corresponding to TAF areas.
    c. Measuring the number of pixels or area corresponding to each of the TAF areas, and subtracting this value from the number of ferrite pixels or the total area of the affected ferrite grain containing the TAF area, thereby obtaining the total area fraction of TAF areas.
    d. Obtaining the total area fraction of the soft phase (soft ferrite), which will be the result of subtracting the fraction of TAF areas from the ferrite fraction.
    e. Quantifying the total area fraction of the hard phase with respect to the total. This will be the sum of the martensite fraction and the affected (hardened) ferrite fraction, obtaining the total hard phase.

f. Optionally, performing calculation of the area fractions based on martensite grain or ferrite sub-phase subgrain sizes, instead of based on the number of pixels of each phase or subphase.

Optionally, for obtaining the EBSD data, a plurality of scans with an increasingly smaller pixel size can be carried out in order to increase the precision in the amount of hardened ferrite identified. After each scan, the number of pixels identified as TAF with respect to the total pixels would be calculated. This would allow the hardened ferrite to be quantified more precisely by repeating the scan until the proportion of ferrite pixels that comply with the conditions for being identified as TAF areas, for each scan, converges to a proportion that corresponds to the amount of hardened ferrite.

In summary, according to the present invention, the use of the EBSD technique and subsequent data processing allows:

- differentiating and quantifying the two existing phases: ferrite (F) and martensite (M), which is already known.
- Identifying and quantifying ferrite sub-phases into soft ferrite and hardened ferrite (TAF areas) systematically, with this being the novel part of the invention. The TAF areas are characterised according to the invention by means of those pixels adjacent to martensite pixels, or as those ferrite pixels adjacent to previously identified TAF pixels and additionally present orientation gradients caused by the adjacent martensite expressed by means of the KAM parameter, with a range of between 0.3 and 2°.

In fact, to date, the following has not been observed in the state of the art:

- Application of strategies of this type based on data obtained by mean of EBSD for the quantification of TAF areas in dual-phase (ferrite-martensite) steels, after differentiation between martensite and ferrite.
- A specific feature of this invention is the selection of the maximum of the KAM parameter as 2° and the minimum of the KAM parameter as 0.3° to identify ferrite areas adjacent to martensite (or areas previously defined as TAF) as TAF areas.
- A preferable feature of this invention is the selection of a fine scan step that is less than or equal to 60 nm (preferably between 50 and 60nm), which allows a more precise characterisation.
- Another preferable feature of this invention is the calculation of the KAM of a pixel with respect to third neighbours (n=3) which allows a more precise characterisation.
- Another even more preferable feature is the selection of a scan that is less than or equal to 60 nm (preferably between 50 and 60 nm), and that the calculation of the KAM of a pixel is with respect to third neighbours (n=3), which allows an even more precise characterisation.

A first embodiment of the invention comprises a method for characterising a recrystallised dual-phase steel sample, which comprises identifying the TAF areas of the sample, comprising the following steps:

- a) carrying out at least one EBSD scan of a surface area of the sample to identify ferrite pixels and martensite pixels;
- b) calculating a KAM deviation with respect to n neighbours for the ferrite pixels;
- c) assigning as a TAF area the ferrite pixels adjacent to at least one martensite pixel,
- d) additionally assigning as a TAF area the ferrite pixels adjacent to at least one ferrite pixel assigned as a TAF area in the previous step and which also have a KAM deviation with respect to n neighbours comprised in a range $[\alpha, \beta]$, where $\alpha \geq 0.3°$ and $\beta \leq 2.0°$.
- e) iterating step d) with respect to all the ferrite pixels adjacent to those previously assigned as a TAF area.

A second embodiment of the invention comprises, in the preceding step c), for assigning TAF areas to the ferrite pixels adjacent to at least one martensite pixel, the additional condition of them having a KAM deviation with respect to n neighbours comprised in a range $[\alpha, \beta]$, where $\alpha \geq 0.3°$ and $\beta \leq 2.0°$, is imposed.

A third embodiment of the invention further comprises quantifying the TAF areas of the sample by means of the following step:

- counting the number of ferrite pixels identified as TAF areas of the sample and comparing it with the total number of pixels of the surface area of the sample.

A fourth embodiment of the invention comprises the preferred definition of n = 3 for calculating the KAM of the pixel according to any of the preceding embodiments, i.e., for calculating the KAM of the pixel with respect to its third neighbours.

A fifth embodiment of the invention defines the pixel size of the EBSD scan as $\leq 200$ nm, preferably $\leq 100$ nm, more preferably $\leq 60$ nm, and even more preferably between 50 and 60 nm, according to any of the preceding embodiments.

A sixth embodiment of the invention defines both jointly: that the pixel size of the EBSD scan is $\leq 60$ nm, and that the calculation of the KAM of a pixel is with respect to third neighbours (n = 3).

A seventh embodiment of the invention comprises carrying out the method for identifying hardened ferrite according to one of the preceding embodiments, by carrying out a plurality of scans with an increasingly smaller pixel size, calculating for each scan the proportion of the number of ferrite pixels that comply with the conditions of the preceding embodiments in order to be considered as TAF areas. This allows quantifying hardened ferrite in a more precise manner; repeating the scan until the proportion of ferrite pixels that comply with said conditions for each scan converges to a proportion which corresponds to the amount of hardened ferrite.

An eighth embodiment of the invention relates to the device for characterising a recrystallised dual-phase steel sample comprising:

- a scanning electron microscope and
- scanning microscope control means for carrying out an EBSD scan;

characterised in that the control means are configured to carry out a method according to one of the preceding embodiments.

A ninth embodiment of the invention relates to the computer software comprising instructions for causing the device of claim 7 to run the steps of the method according to one of the preceding embodiments.

A tenth embodiment of the invention relates to the computer software comprising instructions for processing data obtained previously by means of EBSD such that it runs the steps of characterising the sample of the method, specifically identifying, and optionally quantifying, TAF areas according to one of the preceding embodiments.

An eleventh embodiment of the invention relates to the computer-readable medium having stored therein the computer software of the eighth and/or ninth embodiments.

Another aspect of the present disclosure relates to the use of the quantification of TAF (hardened ferrite) areas in conventional tools for predicting the mechanical properties of DP steels of the state of the art, for the simulation of the micromechanical behaviour of said steels; steels widely used in the form of sheet metal in the automotive industry. The hardening of ferrite appears to cause a change in the mechanical behaviour of the material, which must be taken into account in models for predicting the mechanical properties of steels. This allows determining the mechanical properties of dual-phase steels during industrial forming operations through the improvement of micromechanical models, which allow the mechanical behaviour of these steel grades to be predicted in a more precise manner, and which can be integrated into cold forming simulation software. Thus, this invention contributes to the development of tools for the design of steels with improved mechanical properties.

Lastly, it should be taken into account that this document describes only some embodiments of the invention, therefore, the person skilled in the art will understand that other equivalent or alternative embodiments of the invention, as well as their obvious and equivalent modifications, are also possible. For this reason, the scope of the aspects of the invention should not be limited to the specific embodiments specifically described, and instead should be determined by the appended claims.

**Claims**

1. A method for characterising a recrystallised dual-phase steel sample, which comprises identifying the Transformation Affected Ferrite - TAF - areas of the sample, comprising the following steps:

    - a) carrying out at least one EBSD scan of a surface area of the sample to identify ferrite pixels and martensite pixels;
    - b) calculating a Kernel Average Parameter - KAM - deviation with respect to n neighbours for the ferrite pixels;
    - c) assigning as a TAF area the ferrite pixels adjacent to at least one martensite pixel;
    - d) additionally assigning as a TAF area the ferrite pixels adjacent to at least one ferrite pixel assigned as a TAF area in the previous step and which also have a KAM deviation with respect to n neighbours comprised in a range $[\alpha, \beta]$, where $\alpha \geq 0.3°$ and $\beta \leq 2.0°$;
    - e) iterating step d) with respect to all the ferrite pixels adjacent to those previously assigned as a TAF area.

2. The method according to claim 1, wherein in step c), for assigning TAF areas to the ferrite pixels adjacent to at least one martensite pixel, the additional condition of them having a KAM deviation with respect to n neighbours comprised in a range $[\alpha, \beta]$, where $\alpha \geq 0.3°$ and $\beta \leq 2.0°$, is imposed.

3. The method according to one of claims 1 or 2, which comprises quantifying the TAF areas of the sample by means of the following step:

    - counting the number of ferrite pixels identified as TAF areas of the sample and comparing it with the total number of pixels of the surface area of the sample.

4. The method according to one of claims 1 to 3, wherein n = 3.

5. The method according to one of claims 1 to 4, wherein the pixel size of the EBSD scan is ≤ 200 nm, preferably ≤ 100 nm, more preferably ≤ 60 nm., and even more preferably between 50 and 60 nm.

6. The method according to one of claims 1 to 5, wherein the pixel size of the EBSD scan is ≤ 60 nm, and the calculation of the KAM of a pixel is with respect to third neighbours (n = 3).

7. The method according to one of claims 1 to 6, which comprises carrying out a plurality of scans with an increasingly smaller pixel size, calculating for each scan the proportion of TAF areas.

8. A device for characterising a recrystallised dual-phase steel sample, comprising:

- a scanning electron microscope and
- scanning microscope control means for carrying out an EBSD scan;

**characterised in that**
the control means are configured to carry out the method according to one of claims 1 to 7.

9. Computer software comprising instructions for causing the device of claim 8 to run the steps of the method according to one of claims 1 to 7.

10. Computer software capable of processing data previously obtained by means of EBSD such that it runs steps b-e) of the method according to one of claims 1 to 4.

11. A computer-readable medium having stored therein the computer software of claims 9 and/or 10.


**Patentansprüche**

1. Verfahren zum Charakterisieren einer rekristallisierten zweiphasigen Stahlprobe, das Identifizieren der durch die Umwandlung von Ferrit betroffen - TAF - Flächen der Probe umfasst, umfassend die folgenden Schritte:

- a) Durchführen von zumindest einer EB SD-Abtastung einer Oberflächenfläche der Probe, um Ferritpixel und Martensitpixel zu identifizieren;
- b) Berechnen einer Kerndurchschnittsparameter-, KAM-Abweichung in Bezug auf n Nachbarn für die Ferritpixel;
- c) Zuweisen der Ferritpixel benachbart zu zumindest einem Martensitpixel als TAF-Fläche;
- d) zusätzliches Zuweisen der Ferritpixel benachbart zu zumindest einem Ferritpixel, das in dem vorherigen Schritt als TAF-Fläche zugewiesen wurde und die auch eine KAM-Abweichung in Bezug auf n Nachbarn aufweisen, die in einem Bereich [$\alpha$, $\beta$] umfasst sind, als TAF-Fläche, wobei $\alpha \geq 0{,}3°$ und $\beta \leq 2{,}0°$;
- e) Iterieren von Schritt d) in Bezug auf alle der Ferritpixel benachbart zu denjenigen, die zuvor als TAF-Fläche zugewiesen wurden.

2. Verfahren nach Anspruch 1, wobei in Schritt c) zum Zuweisen von TAF-Flächen zu den Ferritpixeln benachbart zu zumindest einem Martensitpixel die zusätzliche Bedingung, dass sie eine KAM-Abweichung in Bezug auf n Nachbarn aufweisen, die in einem Bereich [$\alpha$, $\beta$] umfasst sind, wobei $\alpha \geq 0{,}3°$ und $\beta \leq 2{,}0°$, auferlegt ist.

3. Verfahren nach einem der Ansprüche 1 oder 2, das Quantifizieren der TAF-Flächen der Probe mittels des folgenden Schrittes umfasst:

- Zählen der Anzahl an Ferritpixeln, die als TAF-Flächen der Probe identifiziert sind, und Vergleichen derselben mit der Gesamtanzahl an Pixeln der Oberflächenfläche der Probe.

4. Verfahren nach einem der Ansprüche 1 bis 3, wobei n = 3.

5. Verfahren nach einem der Ansprüche 1 bis 4, wobei die Pixelgröße der EBSD-Abtastung $\leq 200$ nm, bevorzugt $\leq 100$ nm, bevorzugter $\leq 60$ nm und noch bevorzugter zwischen 50 und 60 nm ist.

6. Verfahren nach einem der Ansprüche 1 bis 5, wobei die Pixelgröße der EBSD-Abtastung $\leq 60$ nm ist und die Berechnung des KAM eines Pixels in Bezug auf dritte Nachbarn (n = 3) ist.

7. Verfahren nach einem der Ansprüche 1 bis 6, das Durchführen einer Vielzahl von Abtastungen mit einer zunehmend kleineren Pixelgröße, Berechnen des Anteils von TAF-Flächen für jede Abtastung umfasst.

8. Vorrichtung zum Charakterisieren einer rekristallisierten zweiphasigen Stahlprobe, umfassend:

- ein Abtastungselektronenmikroskop, und
- Abtastungsmikroskopsteuermittel zum Durchführen einer EBSD-Abtastung;

**dadurch gekennzeichnet, dass**
die Steuermittel konfiguriert sind, um das Verfahren nach einem der Ansprüche 1 bis 7 durchzuführen.

9. Computersoftware, die Anweisungen umfasst, um zu bewirken, dass die Vorrichtung nach Anspruch 8 die Schritte des Verfahrens nach einem der Ansprüche 1 bis 7 ausführt.

10. Computersoftware, die in der Lage ist, Daten, die zuvor mittels EBSD erhalten wurden, zu verarbeiten, sodass sie die Schritte b-e) des Verfahrens nach einem der Ansprüche 1 bis 4 ausführt.

**11.** Computerlesbares Medium, auf dem die Computersoftware nach Anspruch 9 und/oder 10 gespeichert ist.

**Revendications**

**1.** Procédé permettant la caractérisation d'un échantillon d'acier à double phase recristallisé, qui comprend l'identification des zones de ferrite affectée par la transformation - TAF - de l'échantillon, comprenant les étapes suivantes :

- a) la réalisation d'au moins un balayage EBSD d'une surface de l'échantillon pour identifier des pixels de ferrite et des pixels de martensite ;
- b) le calcul d'un écart d'un paramètre moyen du noyau - KAM - par rapport à n voisins pour les pixels de ferrite ;
- c) l'attribution, en tant que zone TAF, des pixels de ferrite adjacents à au moins un pixel de martensite ;
- d) l'attribution, en outre, en tant que zone TAF, des pixels de ferrite adjacents à au moins un pixel de ferrite attribué en tant que zone TAF à l'étape précédente et qui présentent également un écart KAM par rapport à n voisins compris dans une plage $[\alpha, \beta]$, où $\alpha \geq 0,3°$ et $\beta \leq 2,0°$ ;
- e) l'itération de l'étape d) par rapport à tous les pixels de ferrite adjacents à ceux précédemment attribués en tant que zone TAF.

**2.** Procédé selon la revendication 1, dans lequel à l'étape c), pour attribuer des zones TAF aux pixels de ferrite adjacents à au moins un pixel de martensite, la condition supplémentaire, pour eux, d'avoir un écart KAM par rapport à n voisins compris dans une plage $[\alpha, \beta]$, où $\alpha \geq 0,3°$ et $\beta \leq 2,0°$, est imposée.

**3.** Procédé selon l'une des revendications 1 ou 2, qui comprend la quantification des zones TAF de l'échantillon au moyen de l'étape suivante :

- le comptage du nombre de pixels de ferrite identifiés comme des zones TAF de l'échantillon et sa comparaison au nombre total de pixels de la surface de l'échantillon.

**4.** Procédé selon l'une des revendications 1 à 3, dans lequel n = 3.

**5.** Procédé selon l'une des revendications 1 à 4, dans lequel la taille de pixel du balayage EBSD est $\leq 200$ nm, de préférence $\leq 100$ nm, idéalement $\leq 60$ nm, et plus idéalement entre 50 et 60 nm.

**6.** Procédé selon l'une des revendications 1 à 5, dans lequel la taille de pixel du balayage EBSD est $\leq 60$ nm, et le calcul du KAM d'un pixel est par rapport à des troisièmes voisins (n = 3).

**7.** Procédé selon l'une des revendications 1 à 6, qui comprend la réalisation d'une pluralité de balayages avec une taille de pixel de plus en plus petite, calculant pour chaque balayage la proportion de zones TAF.

**8.** Dispositif de caractérisation d'un échantillon d'acier à double phase recristallisé, comprenant :

- un microscope électronique à balayage et
- des moyens de commande de microscope à balayage pour réaliser un balayage EBSD ;

**caractérisé en ce que**
les moyens de commande sont configurés pour réaliser le procédé selon l'une des revendications 1 à 7.

**9.** Logiciel informatique comprenant des instructions pour amener le dispositif de la revendication 8 à exécuter les étapes du procédé selon l'une des revendications 1 à 7.

**10.** Logiciel informatique capable de traiter des données précédemment obtenues au moyen d'EBSD de sorte qu'il exécute les étapes b) à e) du procédé selon l'une des revendications 1 à 4.

**11.** Support lisible par ordinateur présentant, stocké dans celui-ci, le logiciel informatique des revendications 9 et/ou 10.

| M | TAF | TAF | F | F | F | F | F | TAF | TAF | TAF | M | M |
| M | M | TAF | TAF | F | F | F | F | TAF | TAF | M | M | M |
| M | M | M | TAF | TAF | F | F | TAF | TAF | TAF | M | M | M |
| M | M | M | M | TAF | TAF | TAF | TAF | TAF | M | M | M | M |
| M | M | M | M | TAF | TAF | F | F | TAF | M | M | M | M |
| M | M | M | TAF | F | F | F | F | TAF | TAF | M | M | M |
| M | M | M | TAF | F | F | F | F | TAF | M | M | M | M |
| M | M | M | TAF | F | F | F | TAF | TAF | M | M | M | M |
| M | M | TAF | TAF | F | F | F | TAF | M | M | M | M | M |

## Fig. 1

## REFERENCES CITED IN THE DESCRIPTION

**Patent documents cited in the description**

- CN 102735703 A
- CN 103454294 A

- CN 105203438 A

**Non-patent literature cited in the description**

- Analysis of Complex Steel Microstructures by High-Resolution EBSD. **ISATI NERA et al.** JOM: JOURNAL OF METALS. SPRINGER NEW YORK LLC, 26 October 2015, vol. 68, 215-223